# EUROPEAN PATENT SPECIFICATION

(11) **EP 4 363 395 B1**
(45) Date of publication and mention of the grant of the patent: **11.06.2025**
(21) Application number: 22760808.0
(22) Date of filing: 20.06.2022
(51) Int. Cl.: C07C 41/06, C07C 43/13

(54) **METALLOSILICATE CATALYST SOLVENTS**
METALLSILIKATKATALYSATORLÖSUNGSMITTEL
SOLVANTS DE CATALYSEUR À BASE DE MÉTALLOSILICATE

(30) Priority: 28.06.2021 US 202163215564 P
(43) Date of publication of application: 08.05.2024
(73) Proprietor: Dow Global Technologies LLC, Midland, MI 48674 (US)
(72) Inventor: LEE, Wen -Sheng, Midland, Michigan 48674 (US); KU, Sung-Yu, Lake Jackson, Texas 77566 (US); WANG, Le, Freeport, Texas 77566 (US); HUTCHINS, Paul D., Midland, Michigan 48640 (US); PETERSON, Thomas, Midland, Michigan 48640 (US)
(74) Representative: Beck Greener LLP
(86) International application number: PCT/US2022/034131
(87) International publication number: WO 2023/278187

(56) References cited:
- WO-A1-2021/067223

## Description

### BACKGROUND

### Field of the invention

The present disclosure generally relates to metallosilicate catalysts and more specifically to solvents used in conjunction with metallosilicate catalysts.

### Introduction

Production of secondary alcohol ethoxylate surfactants can be carried out by the catalyzed ethoxylation of (poly)alkylene glycol monoalkyl ether ("monoalkyl ether"). The monoalkyl ether is formed from an olefin and a (poly)alkylene glycol using metallosilicate catalysts. Metallosilicate catalysts offer a selectivity for monoalkyl ether of greater than 80% which is advantageous as (poly)alkylene glycol dialkyl ether ("dialkyl ether") is deleterious to properties of the secondary alcohol ethoxylate surfactants. Although the metallosilicate catalysts provide selectivity for monoalkyl ether as high as 85%, The metallosilicate catalysts foul quickly resulting in shorter catalyst lifetime.

Reacting the olefin and the (poly)alkylene glycol to form the monoalkyl ether presents other difficulties as well. For example, the olefin and the (poly)alkylene glycol are generally immiscible in one another. The immiscibility of the two components leads to prolonged times for the reaction to reach greater than 45% olefin conversion because one of the two reactants is not readily available to the catalyst active sites due to the limited solubility. The use of solvents has been disclosed in connection with the formation of monoalkyl ether, but solvents were believed to have no or little effect on the reaction. For example, U.S. Patent number 5,741,948 ("'948 patent") explains that "the reaction between olefin and (poly)alkylene glycol can be conducted in the presence or absence of a solvent" and that the solvent can be "nitromethane, nitroethane, nitrobenzene, dioxane, ethylene glycol dimethyl ether, sulfolane, benzene, toluene, xylene, hexane, cyclohexane, decane, paraffin, etc." As apparent from the optional nature and wide variety of solvents provided in the '948 patent, the presence or type of solvent was not believed to affect the monoalkyl ether reaction.

Accordingly, it would be surprising and unexpected to discover a solvent that when used with metallosilicate catalysts increases the total olefin conversion, decreases the time to reach greater than 45% olefin conversion, and maintains a monoalkyl ether selectivity above 85%.

WO 2021/067223 A1 describes a method for the production of a (poly)alkylene glycol monoalkyl ether by reaction of an olefin, an alcohol, a metallosilicate catalyst and a ester solvent.

### SUMMARY

The present disclosure provides a solvent that when used with metallosilicate catalysts increases the total olefin conversion, decreases the time to reach greater than 45% olefin conversion, and maintains a monoalkyl ether selectivity above 85%.

The present invention is a result of discovering that the above-noted advantages can be achieved by using an aromatic solvent having structure (I): 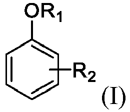 wherein R₁ is selected from the group consisting of a hydrogen or an alkyl group and R₂ is selected from the group consisting of an alkyl group or an alkoxy group. While a solvent may be expected to help solubilize the olefin and the (poly)alkylene glycol, it is surprising and unexpected that the aromatic solvents of structure (I) are able to increase the total olefin conversion, maintain a monoalkyl ether selectivity above 85%, and decrease the time it takes to greater than 45% olefin conversion (i.e., speed up the reaction). Such a result is surprising for at least two reasons. First, although solvents had been used previously in this reaction as demonstrated by the '948 patent, not all solvents produce such advantageous results as demonstrated by the '948 patent's general ambivalence to solvent type or inclusion. Second, the results are surprising because total olefin conversion and monoalkyl ether selectivity typically move inversely to each other yet the use of the solvents having structure (I) increases results for both of these metrics.

The present invention is useful in the production of surfactant precursors.

According to a first feature of the present disclosure, a method for making (poly)alkylene glycol monalkyl ether, comprises the step of contacting an olefin, an alcohol, a metallosilicate catalyst that is an aluminosilicate compound having a crystal lattice that has had one or more metal elements substituted in the crystal lattice for a silicon atom, and a solvent, wherein the solvent comprises structure (I) wherein R₁ is selected from the group consisting of a hydrogen or an alkyl group and R₂ is selected from the group consisting of an alkyl group or an alkoxy group.

According to a second feature of the present disclosure, the alcohol is selected from the group consisting of monoethylene glycol, diethylene glycol, glycerol or combinations thereof.

According to a third feature of the present disclosure, the method comprises the step of generating an alkylene glycol monoalkyl ether.

According to a fourth feature of the present disclosure, the olefin comprises a C₁₂-C₁₄ alpha-olefin.

According to a fifth feature of the present disclosure, the metallosilicate catalyst has a silica to alumina ratio from 10 to 300.

According to a sixth feature of the present disclosure, an alcohol to olefin molar ratio is 0.5 or greater.

According to a seventh feature of the present disclosure, the solvent is from 0.1 wt% to 85 wt% of the combined weight of the olefin, alcohol, and solvent.

According to an eight feature of the present disclosure, R₂ is an alkoxy group.

According to a ninth feature of the present disclosure, R₂ is an alkyl group.

According to a tenth feature of the present disclosure, the solvent is selected from the group consisting of guaiacol, o-cresol, m-cresol, p-cresol, 1,2-dimethoxybenzene, 1,3-dimethoxybenzene, 1,4-dimethoxybenzene and combinations thereof.

### DETAILED DESCRIPTION

As used herein, the term "and/or," when used in a list of two or more items, means that any one of the listed items can be employed by itself, or any combination of two or more of the listed items can be employed. For example, if a composition is described as containing components A, B, and/or C, the composition can contain A alone; B alone; C alone; A and B in combination; A and C in combination; B and C in combination; or A, B, and C in combination.

All ranges include endpoints unless otherwise stated.

Test methods refer to the most recent test method as of the priority date of this document unless a date is indicated with the test method number as a hyphenated two-digit number. References to test methods contain both a reference to the testing society and the test method number. Test method organizations are referenced by one of the following abbreviations: ASTM refers to ASTM International (formerly known as American Society for Testing and Materials); EN refers to European Norm; DIN refers to Deutsches Institut für Normung; and ISO refers to International Organization for Standards.

IUPAC codes describing Crystal structures as delineated by the Structure Commission of the International Zeolite Association refer to the most recent designation as of the priority date of this document unless otherwise indicated.

As used herein, the term weight percent ("wt%") designates the percentage by weight a component is of a total weight of an indicated composition.

As used herein, a "CAS number" is the chemical services registry number assigned by the Chemical Abstracts Service.

### Method

The method of the present invention is directed to the use of solvents in a metallosilicate catalyzed reaction of alcohol and olefin. The method may comprise steps of (a) contacting an olefin, an alcohol, a metallosilicate catalyst and a solvent and (b) generating an alkylene glycol monoalkyl ether.

### Olefin

The olefin used in the method may be linear, branched, acyclic, cyclic, or mixtures thereof. The olefin may have from 5 carbons to 30 carbons (i.e., C₅-C₃₀). The olefin may have 5 carbons or greater, or 6 carbons or greater, or 7 carbons or greater, or 8 carbons or greater, or 9 carbons or greater, or 10 carbons or greater, or 11 carbons or greater, or 12 carbons or greater, or 13 carbons or greater, or 14 carbons or greater, or 15 carbons or greater, or 16 carbons or greater, or 17 carbons or greater, or 18 carbons or greater, or 19 carbons or greater, or 20 carbons or greater, or 21 carbons or greater, or 22 carbons or greater, or 23 carbons or greater, or 24 carbons or greater, or 25 carbons or greater, or 26 carbons or greater, or 27 carbons or greater, or 28 carbons or greater, or 29 carbons or greater, while at the same time, 30 carbons or less, or 29 carbons or less, or 28 carbons or less, or 27 carbons or less, or 26 carbons or less, or 25 carbons or less, or 24 carbons or less, or 23 carbons or less, or 22 carbons or less, or 21 carbons or less, or 20 carbons or less, or 19 carbons or less, or 18 carbons or less, or 17 carbons or less, or 16 carbons or less, or 15 carbons or less, or 14 carbons or less, or 13 carbons or less, or 12 carbons or less, or 11 carbons or less, or 10 carbons or less, or 9 carbons or less, or 8 carbons or less, or 7 carbons or less, or 6 carbons or less.

The olefin may include alkenes such as alpha (α) olefins, internal disubstituted olefins, or cyclic structures (e.g., C₃-C₁₂ cycloalkene). α olefins include an unsaturated bond in the α-position of the olefin. Suitable α olefins may be selected from the group consisting of propylene, 1-butene, 1-hexene, 4-methyl-1-pentene, 1-heptene, 1-octene, 1-decene, 1-dodecene, 1-tetradecene, 1-hexadecene, 1-octadecene, 1-icosene, 1-docosene and combinations thereof. Internal disubstituted olefins include an unsaturated bond not in a terminal location on the olefin. Internal olefins may be selected from the group consisting of 2-butene, 2-pentene, 2-hexene, 3-hexene, 2-heptene, 3-heptene, 2-octene, 3-octene, 4-octene, 2-nonene, 3-nonene, 4-nonene, 2-decene, 3-decene, 4-decene, 5-decene and combinations thereof. Other exemplary olefins may include butadiene and styrene.

Examples of suitable commercially available olefins include NEODENE^{™} 6-XHP, NEODENE^{™} 8, NEODENE^{™} 10, NEODENE^{™} 12, NEODENE^{™} 14, NEODENE^{™} 16, NEODENE^{™} 1214, NEODENE^{™} 1416, NEODENE^{™} 16148 from Shell, The Hague, Netherlands.

### Alcohol

The alcohol utilized in the method may comprise a single hydroxyl group, may comprise two hydroxyl groups (i.e., a glycol) or may comprise three hydroxyl groups. The alcohol may include 1 carbon or greater, or 2 carbons or greater, or 3 carbons or greater, or 4 carbons or greater, or 5 carbons or greater, or 6 carbons or greater, or 7 carbons or greater, or 8 carbons or greater, or 9 carbons or greater, while at the same time, 10 carbons or less, or 9 carbons or less, or 8 carbons or less, or 7 carbons or less, or 6 carbons or less, or 5 carbons or less, or 4 carbons or less, or 3 carbons or less, or 2 carbons or less. The alcohol may be selected from the group consisting of methanol, ethanol, monoethylene glycol, diethylene glycol, propylene glycol, triethylene glycol, polyethylene glycol, monopropylene glycol, dipropylene glycol, tripropylene glycol, polypropylene glycol, 1,3-propanediol, 1,2-butanediol, 2,3-butanediol, 1,4-butanediol, 1,6-hexanediol, 1,4-cyclohexanemethanediol, glycerol and/or combinations thereof. According to various examples, the alcohol is a (poly)alkylene glycol such as monoethylene glycol, diethylene glycol, propylene glycol and triethylene glycol.

A molar ratio of alcohol to olefin in the method may be from be 20:1 or less, or 15:1 or less, or 10:1 or less, or 9:1 or less, or 8:1 or less, or 7:1 or less, or 6:1 or less, or 5:1 or less, or 4:1 or less, or 3:1 or less, or 2:1 or less, or 0.2:1 or less, while at the same time, 0.1:1 or greater, or 1:1 or greater, or 1:2 or greater, or 1:3 or greater, or 1:4 or greater, or 1:5 or greater, or 1:6 or greater, or 1:7 or greater, or 1:8 or greater, or 1:9 or greater, or 1:10 or greater, or 1:15 or greater, or 1:20 or greater. In specific examples, the molar ratio of the alcohol to the olefin may be from 2.0 to 15, or from 2.0 to 8. Molar ratio is calculated by dividing the number of moles of alcohol present by the number of moles of olefin present.

### Solvent

One or more solvents are contacted with the olefin, the alcohol and the metallosilicate catalyst in order to facilitate a chemical reaction. The solvent has structure (I): 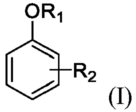 wherein R₁ is selected from the group consisting of a hydrogen or an alkyl group and R₂ is selected from the group consisting of an alkyl group or an alkoxy group.

As used herein, the terms "alkyl" and "alkyl group" refer to a saturated linear, cyclic, or branched hydrocarbon group. Alkyls and alkyl groups also include substituted alkyls. "Substituted alkyl," refers to an alkyl in which one or more hydrogen atom bound to any carbon of the alkyl is replaced by another group such as a halogen, aryl, substituted aryl, cycloalkyl, substituted cycloalkyl, heterocycloalkyl, substituted heterocycloalkyl, halogen, haloalkyl, hydroxy, amino, phosphido, alkoxy, amino, thio, nitro, and combinations thereof. According to various examples, the alkyl may be a linear or branched alkyl having the formula (CH₂)ₘCH₃ wherein m is from 0-10.

As used herein, the term "alkoxy" and "alkoxy group" are defined to mean a functional group containing an alkyl group bonded to an oxygen atom. The alkyl group may be any of the above noted alkyl groups.

The solvent may be selected from the group consisting of guaiacol, o-cresol, m-cresol, p-cresol, 1,2-dimethoxybenzene, 1,3-dimethoxybenzene, 1,4-dimethoxybenzene and combinations thereof. Method may employ one or more solvents. The solvent may include catechol. The solvent may be from 0.1 wt% to 85 wt% of the combined olefin, alcohol and solvent weight. The solvent may be 0.1 wt% or greater, or 0.5 wt% or greater, or 1 wt% or greater, or 5 wt% or greater, or 10 wt% or greater, or 15 wt% or greater, or 20 wt% or greater, or 25 wt% or greater, or 30 wt% or greater, or 35 wt% or greater, or 40 wt% or greater, or 45 wt% or greater, or 50 wt% or greater, or 55 wt% or greater, or 60 wt% or greater, or 65 wt% or greater, or 70 wt% or greater, or 75 wt% or greater, or 80 wt% or greater, while at the same time, 85 wt% or less, or 80 wt% or less, or 75 wt% or less, or 70 wt% or less, 65 wt% or less, or 60 wt% or less, 55 wt% or less, or 50 wt% or less, 45 wt% or less, or 40 wt% or less, 35 wt% or less, or 30 wt% or less, 25 wt% or less, or 20 wt% or less, 15 wt% or less, or 10 wt% or less, 5 wt% or less, or 1 wt% or less, or 0.5 wt% or less of the combined olefin, alcohol and solvent weight.

### Metallosilicate Catalyst

As used herein the term "metallosilicate catalyst" is an aluminosilicate (commonly referred to as a zeolite) compound having a crystal lattice that has had one or more metal elements substituted in the crystal lattice for a silicon atom. The crystal lattice of the metallosilicate catalyst form cavities and channels inside where cations, water and/or small molecules may reside. The substitute metal element may include one or more metals selected from the group consisting of B, Al, Ga, In, Ge, Sn, P, As, Sb, Sc, Y, La, Ti, Zr, V, Cr, Mn, Pb, Pd, Pt, Au, Fe, Co, Ni, Cu, Zn. The metallosilicate catalyst may be substantially free of Hf. According to various examples, the metallosilicate may have a silica to alumina ratio of from 5:1 to 1,500:1 as measured using Neutron Activation Analysis. The silica to alumina ratio may be from 5:1 to 1,500:1, or from 10:1 to 500:1, or from 10:1 to 400:1, or from 10:1 to 300:1 or from 10:1 to 200:1. Such a silica to alumina ratio may be advantageous in providing a metallosilicate catalyst with an appropriate hydrophobic selectivity that adsorb non-polar organic molecules.

The metallosilicate catalyst may have one or more ion-exchangeable cations outside the crystal lattice. The ion-exchangeable cation may include H⁺, Li⁺, Na⁺, Rb⁺, Cs⁺, Mg²⁺, Ca²⁺, Sr²⁺, Ba²⁺, Sc³⁺, Y³⁺, La³⁺, R₄N⁺, R₄P⁺ (where R is H or alkyl).

The metallosilicate catalyst may take a variety of crystal structures. Specific examples of the metallosilicate catalyst structures include MFI (e.g. ZSM-5), MEL (e.g. ZSM-11), BEA (e.g. β-type zeolite), FAU (e.g. Y-type zeolite), MOR (e.g. Mordenite), MTW (e.g. ZSM-12), and LTL (e.g. Linde L), as described using IUPAC codes in accordance with nomenclature by the Structure Commission of the International Zeolite Association.

The crystalline frameworks of metallosilicate catalyst are represented by networks of molecular-sized channels and cages comprised of corner-shared tetrahedral [TO₄] (T=Si or Al) primary building blocks. A negative charge can be introduced onto the framework via the isomorphous substitution of a framework tetravalent silicon by a trivalent metal (e.g., aluminum) atom. The overall charge neutrality is then achieved by the introduction of cationic species compensating for the resulting negative lattice charge. When such a charge-compensation is provided by protons, Brønsted acid sites are formed rendering the resulting H-forms of zeolites strong solid Brønsted acids.

The metallosilicate catalysts may be used in the method in a variety of forms. For example, the metallosilicate catalysts may be powdered (e.g., particles having a longest linear dimension of less than 100 micrometers), granular (e.g., particles having a longest linear dimension of 100 micrometers or greater), or molded articles (e.g., pellets or extrudates) of powdered and/or granular metallosilicate catalysts.

The metallosilicate catalysts may have a surface area of 100 m²/g or greater, or 200 m²/g or greater, or 300 m²/g or greater, or 400 m²/g or greater, or 500 m²/g or greater, or 600 m²/g or greater, or 700 m²/g or greater, or 800 m²/g or greater, or 900 m²/g or greater, while at the same time, 1000 m²/g or less, or 900 m²/g or less, or 800 m²/g or less, or 700 m²/g or less, or 600 m²/g or less, or 500 m²/g or less, or 400 m²/g or less, or 300 m²/g or less, or 200 m²/g or less. Surface area is measured according to ASTM D4365-19.

Metallosilicate catalysts can be synthesized by hydrothermal synthesis methods. For example, the metallosilicate catalysts can be synthesized from heating a composition comprising a silica source (e.g., silica sol, silica gel, and alkoxysilanes), a metal source (e.g., metal sulfates, metal oxides, metal halides, etc.), and a quaternary ammonium salt such as a tetraethylammonium salt or tetrapropylammonium to a temperature of about 100°C to about 175°C until a crystal solid forms. The resulting crystal solid is then filtered off, washed with water, and dried, and then calcined at a temperature form 350°C to 600°C.

Examples of suitable commercially available, metallosilicate catalysts include CP814E, CP814C, CP811C-300, CBV 712, CBV 720, CBV 760, CBV 2314, CBV 10A from ZEOLYST INTERNATIONAL^{™} of Conshohocken, PA.

### Generating Monoalkyl Ether

Contacting the olefin, alcohol, metallosilicate catalyst and solvent in the generation of an alkylene glycol monoalkyl ether. The chemical reaction between the olefin and the alcohol is catalyzed by the metallosilicate catalyst in a reactor to generate the monoalkyl ether. Various monoalkyl ethers may be produced for different applications by varying which olefin is utilized and/or by varying which alcohol is utilized. Monoalkyl ether are utilized for a number of applications such as solvents, surfactants, and chemical intermediates, for instance.

The reaction of the olefin and the alcohol may take place at from 50°C to 300°C or from 100°C to 200°C. In a specific example the reaction may be carried out at 150°C. Reaction of the olefin and the alcohol may be carried out in a batch reactor, continuous reactor, or fixed-bed reactor. In operation of the chemical reaction, the Brønsted acid sites of the metallosilicate catalyst catalyze the etherification of the olefin to the alcohol through an addition type reaction. The reaction of the olefin and the alcohol produces the monoalkyl ether.

The addition reaction of the olefin to the glycol may form not only monoalkyl ether but also the dialkyl ether. The metallosilicate catalyst may exhibit a selectivity to produce alkylene monoalkyl ether, but not dialkyl ether. The monoalkyl ether selectivity may be 70% or greater, or 75% or greater, or 80% or greater, or 85% or greater, or 90% or greater, or 95% or greater or 99% or greater, while at the same time, 100% or less, or 95% or less, or 90% or less, or 85% or less, or 80% or less, or 75% or less. The dialkyl ether selectivity may be 0% or greater, or 2% or greater, or 4% or greater, or 6% or greater, or 8% or greater, or 10% or greater, or 12% or greater, or 14% or greater, or 16% or greater, or 18% or greater, while at the same time, 20% or less, or 18% or less, or 16% or less, or 14% or less, or 12% or less, or 10% or less, or 8% or less, or 6% or less, or 4% or less, or 2% or less.

A monoalkyl ether yield is calculated by multiplying the amount of olefin conversion by the monoalkyl ether selectivity. The alkylene glycol monoalkyl ether yield may be 10% or greater, or 15% or greater, or 20% or greater, or 25% or greater, or 30% or greater, or 35% or greater, while at the same time, 40% or less, or 35% or less, or 30% or less, or 25% or less, or 20% or less, or 15% or less. Monoalkyl ether yield is a measure of the catalytic activity and selectivity and is a good measure of the production rate of the metallosilicate catalyst.

During the reaction of the olefin and the alcohol, the catalyst becomes fouled and results it the deactivating (i.e., lost etherification activity >90%) the catalyst within hours.

### Examples

Catalyst is a metallosilicate catalysts defined by a BEA structure and having a silica to alumina ratio of 25:1 and a surface area of 680 m²/g, that is commercially available as CP814E from ZEOLYST INTERNATIONAL^{™} of Conshohocken, PA.

Olefin is 1-Dodecene that is commercially available as NEODENE^{™} 12 from the SHELL^{™} group of The Hague, Netherlands.

Monoethylene Glycol ("MEG") is liquid anhydrous ethylene glycol having a CAS Number of 107-21-1 and is commercially available from SIGMA ALDRICH^{™} St. Louis, Missouri.

Diglyme is bis(2-methoxyethyl) ether having a CAS number of 111-96-6 and is commercially available from SIGMA ALDRICH^{™} St. Louis, Missouri.

Guaiacol is 2-Methoxyphenol having a CAS number of 90-05-1 and is commercially available from SIGMA ALDRICH^{™} St. Louis, Missouri.

Cresol is an 8/3 weight ratio mixture of M-cresol and P-cresol. M-Cresol is 1-Hydroxy-3-methylbenzene having a CAS number of 108-39-4 and is commercially available from SIGMA ALDRICHTM St. Louis, Missouri. P-cresol is 4-Methylphenol having a CAS number of 106-44-5 and is commercially available from SIGMA ALDRICH^{™} St. Louis, Missouri.

DMB is 1,4-dimethoxybenzene having a CAS number of 150-78-7 and is commercially available from SIGMA ALDRICH^{™} St. Louis, Missouri.

### Test Methods

### Gas Chromatography Samples

Prepare gas chromatography samples by mixing 100 µL of the example with 10 mL of gas chromatography solution that was prepared by addition of 1 mL of hexadecane in 1 L of ethyl acetate. Analyze the sample using an Agilent 7890B gas chromatography instrument. Determine the total amount of 1-dodecene derived species, which includes monoalkyl ether, dialkyl ether and 2-dodecanol, total amount of dodecene, which includes 1-dodecene and all non 1-dodecene other C₁₂ isomers. Table 1 provides the relevant gas chromatography instrument parameters.

**Table 1:**

| | |
|---|---|
| Chromatograph: | Agilent 7890 Series GC |
| Column: | Agilent HP88, 100 m x 0.25 mm x 0.20 um |
| Detector | FID |
| Oven: | 50 °C - 7min - 6 °C/min - 260 °C - 1 min |
| Injector: | 250 °C |
| Detector: | 300 °C |
| Carrier: | Helium 2.0 mL/min constant flow mode |
| Split ratio: | 10 |
| Make-Up: | Nitrogen 25mL/min |
| Air: | 400 mL/min |
| Hydrogen: | 40 mL/min |
| Inlet Liner: | Restek PN 23305.5 Sky Precision Liner with wool |
| Sample Size: | 1 µL |
| GC vial rinsing solvent: | ethyl acetate |

### Time-On-Stream ("TOS")

Calculate the TOS of the catalyst by measuring the total time the catalyst has been in contact with the monoethylene glycol, dodecene, catalyst, solvent and products at temperatures above 60°C.

### Olefin Conversion

Calculate the percent olefin conversion by dividing the total amount of dodecene derived species by the summation of total amount of dodecene derived species and the amount of dodecene. Multiply the quotient by 100.

### Monoalkyl Ether Selectivity

Calculate the percent monoalkyl ether selectivity by dividing the total amount of monoalkyl ether by the total amount of dodecene derived species. Multiply the quotient by 100.

### Monoether Yield

Calculate the monoalkyl ether yield by multiplying the olefin conversion value by the monoalkyl ether selectivity value divided by 100.

### Sample Preparation

The etherification reaction was carried out in either a Parr reactor or a vial reactor. Regardless of reactor type, the reactants used were 1-dodecene, ethylene glycol, desired solvent(s) and the catalyst. The Parr reactor was a 300 mL reactor with a heating jacket and controller. The Parr reactor used a pitch blade impeller for agitation. For the vial reactor runs, a 40 mL vial had the reactants added along with a V&P Scientific^{™} stir bar. The vial reactors were then placed in a device that both heated the vial reactor to the desired temperature as well as produced stirring in a tumbling style by the stir bar. Both Parr and Vial reactors were run at 135°C.

**Table 2:**

| Example | Reactor type | **Olefin** (g) | **MEG** (g) | Catalyst (g) | Solvent (g) |
|---|---|---|---|---|---|
| CE1 | Parr | 50 | 100 | 7.5 | 0 |
| CE2 | Parr | 30 | 150 | 7.5 | 0 |
| IE7 | Parr | 10 | 10 | 7.5 | 100 Diglyme |
| | | | | | 100 Guaiacol |
| CE4 | Vial | 2 | 2 | 7.5 | 10 Diglyme |
| IE1 | Parr | 10 | 10 | 7.5 | 200 Guaiacol |
| IE2 | Parr | 10 | 10 | 7.5 | 200 Cresol |
| IE3 | Vial | 2 | 2.1 | 0.75 | 11 Cresol |
| IE4 | Vial | 1 | 1 | 0.75 | 10 DMB |
| IE5 | Vial | 1 | 1 | 0.75 | 10 Guaiacol |
| IE6 | Vial | 1 | 1 | 0.75 | 10.2 Cresol |

### Results

Below are the results after the inventive and comparative examples were reacted for the indicated time. Table 3 provides the time on stream olefin conversion ("conversion"), monoether selectivity ("selectivity"), and monoether yield ("yield") for each of CE1, CE2 and CE4 and IE1-7.

**Table 3**

| Ex. | Olefin:MEG Ratio | Solvent Type | TOS (hrs) | Conversion (%) | Selectivity (%) | Yield (%) |
|---|---|---|---|---|---|---|
| CE1 | 2:1 | None | 3.00 | 10.90 | 95.29 | 10.39 |
| | | | 6.00 | 18.31 | 92.82 | 16.99 |
| | | | 9.00 | 23.96 | 90.98 | 21.80 |
| | | | 24.00 | 35.99 | 82.65 | 29.75 |
| | | | 39.00 | 40.09 | 79.90 | 32.03 |
| CE2 | 5:1 | None | 3.00 | 14.80 | 93.84 | 13.88 |
| | | | 6.00 | 25.70 | 90.41 | 23.23 |
| | | | 9.00 | 33.19 | 87.50 | 29.04 |
| | | | 24.00 | 48.36 | 77.98 | 37.71 |
| | | | 39.00 | 47.56 | 64.94 | 30.89 |
| IE7 | 1:1 | Diglyme + guaiacol | 2.00 | 17.53 | 93.48 | 16.39 |
| | | | 4.00 | 22.95 | 91.05 | 20.89 |
| | | | 6.00 | 26.21 | 92.09 | 24.14 |
| | | | 8.00 | 24.73 | 92.58 | 22.89 |
| IE1 | 1:1 | Guaiacol | 1.00 | 33.64 | 90.27 | 30.37 |
| | | | 2.00 | 45.36 | 89.99 | 40.82 |
| | | | 3.00 | 48.35 | 91.22 | 44.10 |
| | | | 4.00 | 47.23 | 92.14 | 43.51 |
| | | | 6.00 | 40.51 | 94.62 | 38.33 |
| | | | 8.00 | 37.05 | 92.43 | 34.25 |
| IE2 | 1:1 | Cresol | 2.00 | 51.81 | 96.11 | 49.79 |
| | | | 4.00 | 42.78 | 97.76 | 41.82 |
| | | | 6.00 | 39.29 | 89.55 | 35.18 |
| | | | 8.00 | 41.33 | 88.23 | 36.47 |
| CE4 | 1:1 | Diglyme | 1.00 | 3.91 | 100.00 | 3.91 |
| | | | 3.00 | 13.14 | 97.24 | 12.78 |
| | | | 5.00 | 19.63 | 96.57 | 18.96 |
| | | | 7.50 | 25.14 | 95.56 | 24.03 |
| | | | 10.00 | 28.60 | 94.92 | 27.15 |
| | | | 12.00 | 30.86 | 94.65 | 29.21 |
| | | | 15.00 | 33.33 | 93.85 | 31.28 |
| IE3 | 1.05:1 | Cresol | 1.00 | 30.89 | 93.67 | 28.94 |
| | | | 3.00 | 54.39 | 90.69 | 49.32 |
| | | | 5.00 | 60.57 | 90.31 | 54.71 |
| | | | 7.50 | 60.04 | 90.82 | 54.53 |
| | | | 10.00 | 58.58 | 92.04 | 53.91 |
| IE4 | 1:1 | DMB | 1.00 | 24.19 | 91.72 | 22.19 |
| | | | 3.00 | 47.49 | 87.14 | 41.38 |
| | | | 5.00 | 52.61 | 87.76 | 46.17 |
| | | | 7.50 | 47.92 | 89.99 | 43.12 |
| | | | 10.00 | 44.55 | 91.12 | 40.60 |
| IE5 | 1:1 | Guaiacol | 1.00 | 34.79 | 91.60 | 31.87 |
| | | | 3.00 | 51.97 | 90.22 | 46.89 |
| | | | 5.00 | 52.69 | 91.03 | 47.96 |
| | | | 7.50 | 50.89 | 91.61 | 46.62 |
| | | | 10.00 | 52.57 | 91.97 | 48.34 |
| IE6 | 1:1 | Cresol | 1.00 | 47.98 | 92.39 | 44.33 |
| | | | 3.00 | 44.58 | 95.71 | 42.67 |
| | | | 5.00 | 38.62 | 97.63 | 37.71 |
| | | | 7.50 | 33.36 | 98.83 | 32.97 |
| | | | 10.00 | 32.71 | 98.52 | 32.22 |

Referring now to Table 3, as can be seen the use of a solvent having structure (I) enables increased total olefin conversion, maintenance of a monoalkyl ether selectivity above 85%, and a reduction the TOS to reach greater than 45% olefin conversion (i.e., increasing the speed of the reaction). CE1 and CE2 demonstrate that when no solvent is used the reaction takes 24 hours or more to reach greater than 45% olefin conversion. Further, CE1 and CE2 demonstrate that without a solvent the selectivity of the reaction decreases with time (i.e., to below 75%) meaning more undesirable reaction products are being produced. CE4 demonstrates that while diglyme (i.e., a solvent not having Structure (I)) can speed the reaction to reach total olefin conversion faster and maintain selectivity, the total olefin conversion is unacceptably low (i.e., <35%). Contrary to the comparative examples, IE1-IE6 all demonstrate the ability to increase the total olefin conversion, maintain a monoalkyl ether selectivity above 85%, and reduce the TOS to reach the peak total olefin conversion. For example, each of IE1-IE6 are able to achieve total olefin conversions of from 48% to 60% whereas the greatest olefin conversion of CE1-CE4 is 48%. Further, the monoether selectivity with no solvent decreases overtime to a low of 64% (CE2) where as IE1-IE4 are able to maintain selectivity as great as 87% over the life of the reaction. Finally, IE1-IE6 are able to offer greater than 45% olefin conversion within less than 10 hours whereas CE1, CE2 and CE4 all require greater than 10 hours TOS to achieve peak olefin conversion which in many cases is not greater than 45%.

## Claims

1. A method for making (poly)alkylene glycol monalkyl ether, comprising the step:
contacting an olefin, an alcohol, a metallosilicate catalyst that is an aluminosilicate compound having a crystal lattice that has had one or more metal elements substituted in the crystal lattice for a silicon atom, and a solvent, wherein the solvent comprises structure (I): 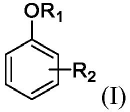 wherein R₁ is selected from the group consisting of a hydrogen or an alkyl group and R₂ is selected from the group consisting of an alkyl group or an alkoxy group.

2. The method of claim 1, wherein the alcohol is selected from the group consisting of monoethylene glycol, diethylene glycol, glycerol or combinations thereof.

3. The method of claim 1, further comprising the step of:
generating an alkylene glycol monoalkyl ether.

4. The method of any one of claims 1-3, wherein the olefin comprises a C₁₂-C₁₄ alpha-olefin.

5. The method of any of claims 1-4, wherein the metallosilicate catalyst has a silica to alumina ratio from 10 to 300.

6. The method of any of claims 1-5, wherein an alcohol to olefin molar ratio is 0.5 or greater.

7. The method of any one of claims 1-5, wherein the solvent is from 0.1 wt% to 85 wt% of the combined weight of the olefin, alcohol, and solvent.

8. The method of any of claims 1-7, wherein R₂ is an alkoxy group.

9. The method of any of claims 1-7, wherein R₂ is an alkyl group.

10. The method of any of claims 1-7, wherein the solvent is selected from the group consisting of guaiacol, o-cresol, m-cresol, p-cresol, 1,2-dimethoxybenzene, 1,3-dimethoxybenzene, 1,4-dimethoxybenzene and combinations thereof.

## Patentansprüche

1. Verfahren zum Herstellen von (Poly)alkylenglykolmonalkylether, umfassend den Schritt:
Inberührungbringen eines Olefins, eines Alkohols, eines Metallsilikatkatalysators, der eine Alumosilikatverbindung ist, die ein Kristallgitter aufweist, wobei in dem Kristallgitter ein oder mehrere Metallelemente durch ein Siliziumatom ersetzt wurden, und eines Lösungsmittels, wobei das Lösungsmittel die Struktur (I) umfasst: 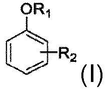
wobei R₁ aus der Gruppe ausgewählt ist, bestehend aus einem Wasserstoff oder einer Alkylgruppe und R₂ aus der Gruppe ausgewählt ist, bestehend aus einer Alkylgruppe oder einer Alkoxygruppe.

2. Verfahren nach Anspruch 1, wobei der Alkohol aus der Gruppe ausgewählt ist, bestehend aus Monoethylenglykol, Diethylenglykol, Glycerin oder Kombinationen davon.

3. Verfahren nach Anspruch 1, ferner umfassend den Schritt:
Erzeugen eines Alkylenglykolmonoalkylethers.

4. Verfahren nach einem der Ansprüche 1 bis 3, wobei das Olefin ein C₁₂-C₁₄-Alpha-Olefin ist.

5. Verfahren nach einem der Ansprüche 1 bis 4, wobei der Metallsilikatkatalysator ein Verhältnis von Siliciumdioxid zu Aluminiumoxid von 10 bis 300 aufweist.

6. Verfahren nach einem der Ansprüche 1 bis 5, wobei ein Verhältnis von Alkohol zu Olefin 0,5 oder mehr beträgt.

7. Verfahren nach einem der Ansprüche 1 bis 5, wobei das Lösungsmittel von 0,1 Gew.-% bis 85 Gew.-% des Gesamtgewichts des Olefins, Alkohols und Lösungsmittels ausmacht.

8. Verfahren nach einem der Ansprüche 1 bis 7, wobei R₂ eine Alkoxygruppe ist.

9. Verfahren nach einem der Ansprüche 1 bis 7, wobei R₂ eine Alkylgruppe ist.

10. Verfahren nach einem der Ansprüche 1 bis 7, wobei das Lösungsmittel aus der Gruppe ausgewählt ist, bestehend aus Guajakol, o-Kresol, m-Kresol, p-Kresol, 1,2-Dimethoxybenzol, 1,3-Dimethoxybenzol, 1,4-Dimethoxybenzol und Kombinationen davon.

## Revendications

1. Procédé de fabrication d'un éther monalkylique de (poly)alkylène glycol, comprenant l'étape consistant à :
mettre en contact une oléfine, un alcool, un catalyseur de métallosilicate qui est un composé d'aluminosilicate ayant un réseau cristallin dont un ou plusieurs éléments métalliques ont été substitués dans le réseau cristallin par un atome de silicium, et d'un solvant, dans lequel le solvant comprend la structure (1) : 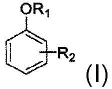
où R₁ est choisi dans le groupe constitué d'un hydrogène ou d'un groupe alkyle et R₂ est choisi dans le groupe constitué d'un groupe alkyle ou d'un groupe alcoxy.

2. Procédé selon la revendication 1, dans lequel l'alcool est choisi dans le groupe constitué de monoéthylène glycol, diéthylène glycol, et combinaisons de ceux-ci.

3. Procédé selon la revendication 1 comprenant l'étape consistant à :
générer un éther monoalkylique d'alkylène glycol.

4. Procédé selon l'une quelconque des revendications 1 à 3, dans lequel l'oléfine comprend une alpha oléfine en C₁₂-C₁₄.

5. Procédé selon l'une quelconque des revendications 1 à 4, dans lequel le catalyseur à base de métallosilicate présente un rapport silice/alumine allant de 10 à 300.

6. Procédé selon l'une quelconque des revendications 1 à 5, dans lequel un rapport molaire entre l'alcool et l'oléfine est égal ou supérieur à 0,5.

7. Procédé selon l'une quelconque des revendications 1 à 5, dans lequel le solvant représente de 0,1 % en poids à 85 % en poids du poids combiné de l'oléfine, de l'alcool et du solvant.

8. Composition selon l'une quelconque des revendications 1 à 7, dans laquelle R₂ est un groupe alcoxy.

9. Composition selon l'une quelconque des revendications 1 à 7, dans laquelle R₂ est un groupe alkyle.

10. Procédé selon l'une quelconque des revendications 1 à 7, dans lequel le solvant est choisi dans le groupe constitué de gaïacol, o-crésol, m-crésol, p-crésol, 1,2-diméthoxybenzène, 1,3-diméthoxybenzène, 1,4-diméthoxybenzène et des combinaisons de ceux-ci.
